(19) Europäisches Patentamt
European Patent Office
Office européen des brevets

(11) Publication number: **0 562 497 A1**

(12) **EUROPEAN PATENT APPLICATION**

(21) Application number: **93104592.6**

(22) Date of filing: **20.03.93**

(51) Int. Cl.5: **C07C 401/00**, A61K 31/59, C07J 9/00, C07J 71/00

(30) Priority: **27.03.92 JP 71007/92**
**28.12.92 JP 347480/92**

(43) Date of publication of application:
**29.09.93 Bulletin 93/39**

(84) Designated Contracting States:
**CH DE FR GB IT LI NL**

(71) Applicant: **NISSHIN FLOUR MILLING CO., LTD.**
**19-12, Nihonbashi-koamicho**
**Chuo-ku, Tokyo(JP)**

(72) Inventor: **Tachibana, Yoji**
**5024-742, Kasahata, Kawagoe-shi**
**Saitama-ken(JP)**
Inventor: **Yokoyama, Shinji**
**23-16, Midoriagaoka 2-chome, Ohimachi**
**Iruma-gun, Saitama-ken(JP)**
Inventor: **Tejima, Tsuyoshi**
**23-16, Midoriagaoka 2-chome, Ohimachi**
**Iruma-gun, Saitama-ken(JP)**
Inventor: **Okamoto, Yasushi**
**3-3-3, Shakujiidai, Nerima-ku**
**Tokyo(JP)**
Inventor: **Hongyo, Takayuki**
**23-16, Midorigaoka 2-chome, Ohimachi**
**Iruma-gun, Saitama-ken(JP)**

(74) Representative: **Türk, Gille, Hrabal, Leifert**
**Brucknerstrasse 20**
**D-40593 Düsseldorf (DE)**

(54) **1 alpha-hydroxy vitamins D7 and D4' processes for the preparation thereof and pharmaceutical compositions.**

(57) Disclosed are new 1α-hydroxy vitamin $D_7$ of formula (I) and 1α-hydroxy vitamin $D_4$ of formula (II) which are useful as active ingredients of pharmaceutical compositions for the treatment of osteoporosis.

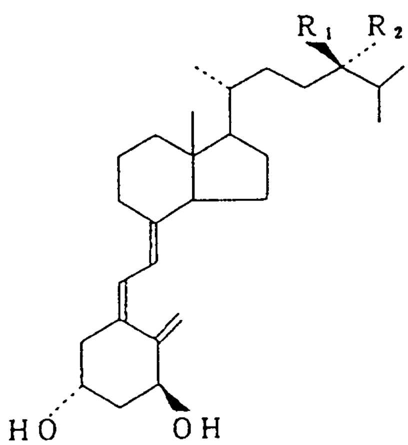

(I) : $R_1 = CH_3$, $R_2 = H$

(II) : $R_1 = H$, $R_2 = CH_3$

Those new compounds are synthesized starting from (22E)-5α,8α-(4-phenyl-1,2-urazolo)-6,22-ergostadien-3β-ol 3β-tert-butyl-dimethylsilyl ether and through ten process steps.

EP 0 562 497 A1

## FIELD OF THE INVENTION

This invention relates to new 1α-hydroxy vitamin $D_7$, 1α-hydroxy vitamin $D_4$, new intermediates used in the synthesis thereof and processes for the preparation thereof. The invention also relates to pharmaceutical compositions which comprise as an active ingredient new 1α-hydroxy vitamin $D_7$ or 1α-hydroxy vitamin $D_4$, which possesses improved activity to increase a mineral bone density.

## BACKGROUND OF THE ART

The active forms of vitamin $D_3$, 1α,25-dihydroxy vitamin $D_3$ and 1α-hydroxy vitamin $D_3$ are known to be effective in the regulation of calcium metabolism including intestinal calcium absorption, intestinal calcium transport, bone calcium mobilization and bone calcification. These compounds exhibiting high activity in vivo have been used as a therapeutic agent for osteoporosis. However, an excessive intake of such compounds may cause side effects such as hypercalcemia because of its strong biological activity.

An investigation has been undertaken to synthesize the active forms of vitamin D derivatives having bone mineral density-enhancing activity equal to or more than the active forms of vitamin $D_3$ and further having reduced side effects and lower toxicity. It has been reported that (24R)- and (24S)-22,23-dihydro-1α,25-dihydroxy vitamin $D_2$ represented by formula (A) or (B) have lower toxicity and furthermore bone mineral density-enhancing activity of the same level as active forms of vitamin $D_3$ (Biochim. Biophys. Acta. 1091 (1991) 188-192). The analogues of such compounds are expected to possess an interesting pharmacological activity in association with the active forms of vitamins $D_3$ and $D_2$. However, 1α-hydroxy vitamin $D_7$ and 1α-hydroxy vitamin $D_4$, i.e., the compounds in which hydrogen substitutes a hydroxyl group at 25 position of the compound of formula (A) or (B) are not known because of difficulty and complexity in the synthesis.

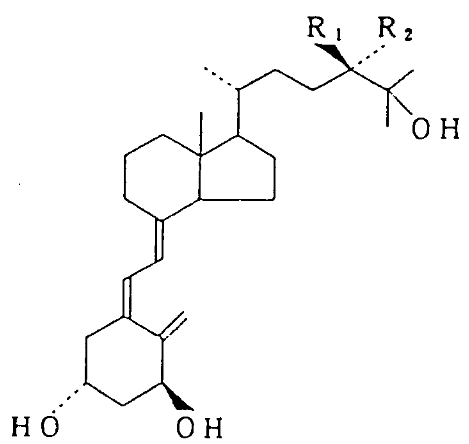

(A) : $R_1$=Me, $R_2$=H

(B) : $R_1$=H, $R_2$=Me

## DETAILED DESCRIPTION OF THE INVENTION

The present inventors were successful in the synthesis of 1α-hydroxy vitamin $D_7$ and 1α-hydroxy vitamin $D_4$ and discovered that they possess reduced side effects as well as more improved activity to increase a mineral bone density and to induce a cell differentiation as compared with known active forms of vitamin D.

The term "activity to increase a mineral bone density" as used herein refers to the activity to prevent bone loss or restore lost bone.

Thus the invention provides new 1α-hydroxy vitamin $D_7$ of formula (I) and 1α-hydroxy vitamin $D_4$ of formula (II).

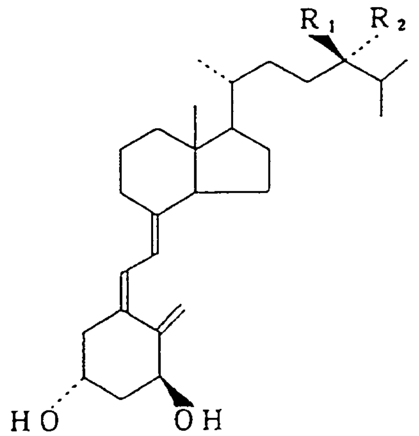

(I) : $R_1$=Me, $R_2$=H

(II) : $R_1$=H, $R_2$=Me

For studying new compounds of the invention, it was necessary to develop processes for their production. One alpha-hydroxy vitamin $D_7$ was synthesized for the first time and in the course of that synthesis new intermediates were also produced.

Thus the invention also provides processes for the preparation of new 1α-hydroxy vitamin $D_7$ of formula (I), 1α-hydroxy vitamin $D_4$ of formula (II) and new intermediates used in the synthesis thereof.

The synthesis of 1α-hydroxy vitamin $D_7$ (formula I) and 1α-hydroxy vitamin $D_4$ (formula II) is accomplished according to the method shown for example in process scheme I.

PROCESS SCHEME I

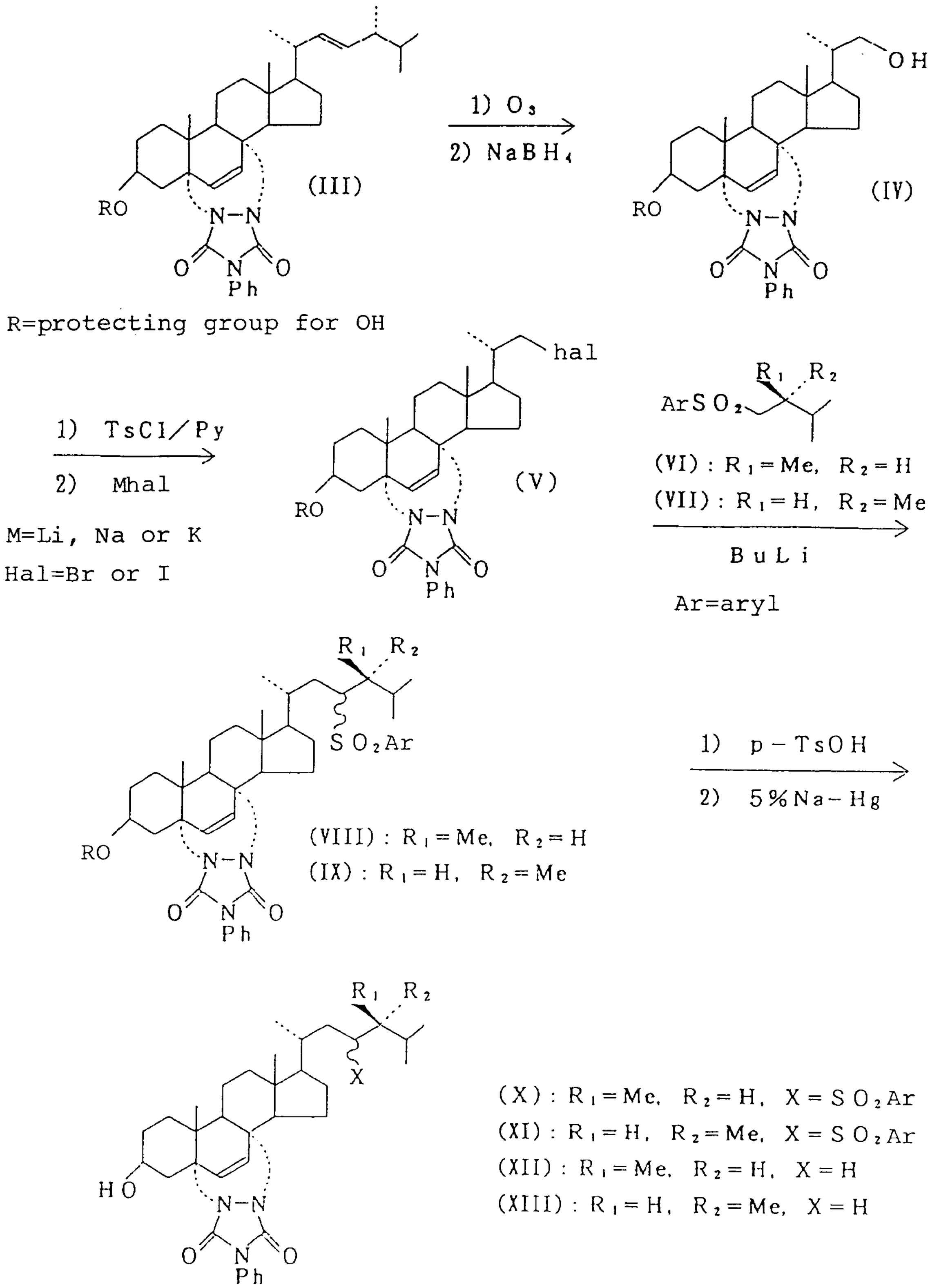

PROCESS SCHEME I (continued)

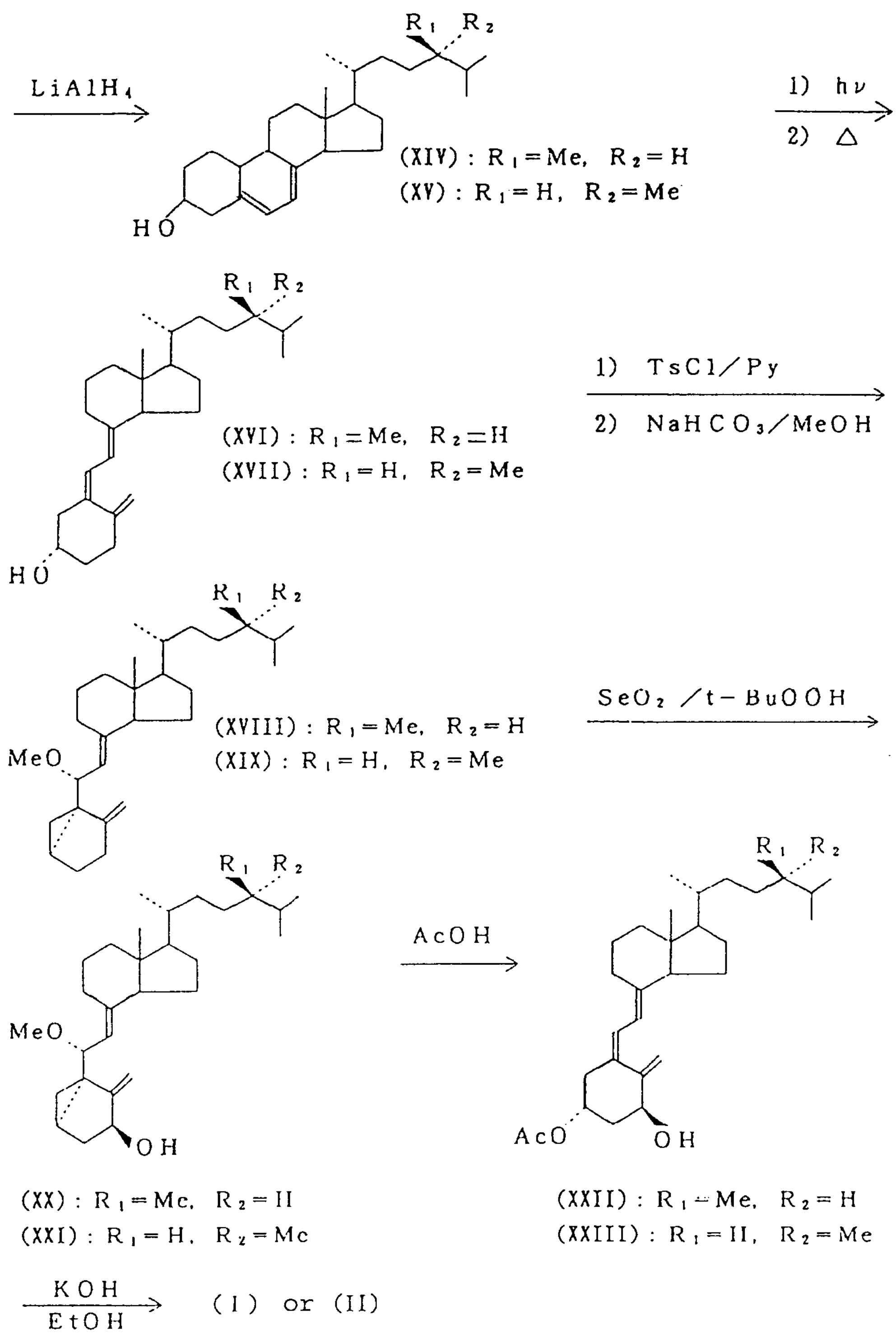


(XIV) : $R_1$=Me, $R_2$=H
(XV) : $R_1$=H, $R_2$=Me

(XVI) : $R_1$=Me, $R_2$=H
(XVII) : $R_1$=H, $R_2$=Me

(XVIII) : $R_1$=Me, $R_2$=H
(XIX) : $R_1$=H, $R_2$=Me

(XX) : $R_1$=Me, $R_2$=H
(XXI) : $R_1$=H, $R_2$=Me

(XXII) : $R_1$=Me, $R_2$=H
(XXIII) : $R_1$=H, $R_2$=Me

(I) or (II)

PROCESS SCHEME II

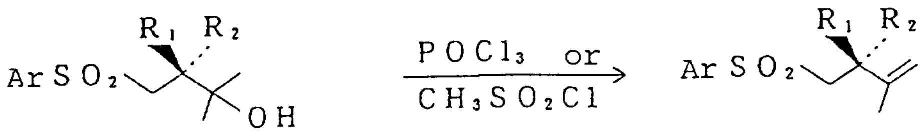


(XXIV) : $R_1 = CH_3$, $R_2 = H$
(XXV) : $R_1 = H$, $R_2 = CH_3$

(XXVI) : $R_1 = CH_3$, $R_2 = H$
(XXVII) : $R_1 = H$, $R_2 = CH_3$

$H_2/Pd-C$

(VI) or (VII)

As shown in process scheme I, the synthesis starts from the compound of formula (III) in which the 5,7-diene and the hydroxyl group at 3-position of (22E)-5,7,22-ergostatrien-3$\beta$-ol are protected, respectively with 4-phenyl-1,2,4-triazoline-3,5-dione (PTAD) and with suitable protecting group conventionally used for the protection of hydroxyl group, e.g. tert-butyldimethylsilyl, trimethylsilyl, acetyl group or the like. The compound (III) is subjected to ozone oxidation to form the 22-aldehyde compound which is then reduced (e.g., with $NaBH_4$) to the 22-alcohol compound of formula (IV). The ozone oxidation is carried out at a temperature below -20°C in an inert solvent such as dichloromethane, dichloroethane, chloroform or the like, which is used in 5 to 50 times the amount of the compound (III). In that case, 1 to 5% of pyridine may be added to the compound (III). The reduction is conducted at a temperature between -50°C and room temperature. In that case, $NaBH_4$ is used in 1 to 5 times the amount of compound (III). The compounds (III) and (IV) are separated and purified by a silica gel chromatography and the compound (III) thus recovered can be recycled.

The compound (IV) is subjected to tosylation followed by halogenation to form the 22-halo compound of formula (V). The tosylation is carried out at a temperature of 0°C to 30°C for 1 to 20 hours in suitable solvents such as pyridine, triethylamine, diisopropylamine or the like. For example, tosyl chloride is used in this reaction in 1.0 to 10 times the amount of the compound (IV). The solvents are used in 5 to 20 times the amount of the compound (IV). After conventional working up, the reaction product is used for the subsequent reaction. The halogenation is carried out at a temperature between room temperature and boiling point of the solvent for 1 to 10 hours in suitable solvents such as acetone, methyl ethyl ketone, dimethylformamide or the like. The solvents are used in 5 to 20 times the amount of the compound (IV). For example, an alkali metal halide such as NaI, NaBr, KI, KBr or the like can be used as a halogenating agent in the amount of 1.0 to 10 times that of the compound (IV). Purification of the reaction product is conducted by crystallization from hexane, ethyl acetate or the like.

The 22-halo compound (V) is condensed with a sulfone derivative of formula (VI) or (VII) under a basic condition e.g., using n-butyl lithium, lithium diisopropylamide (LDA), sodium hydride or the like to form the compound of formula (VIII) or (IX). The sulfone derivative is used in 1 to 5 times the amount of the compound (V). This condensation reaction is carried out at a temperature of -30°C to 30°C for 0.5 to 5 hours in suitable solvents such as THF, ether or the like which are used in 5 to 20 times the amount of the compound (V), preferably in a gas stream such as $N_2$ and Ar and if desired, in the presence of hexamethylphosphoric triamide (HMPA) or 1,3-dimethyl-2-imidazolidinone.

The protecting group at 3$\beta$ position is removed from the compound (VIII) or (IX) to form the diol compound of formula (X) or (XI). This reaction is carried out at a temperature of 0 to 30°C under an acid condition in suitable proton solvents. The acids such as p-toluene sulfonic acid, pyridinium-p-toluene sulfonic acid or the like are used in 0.01 to 0.5 times the amount of the compound (VIII) or (IX). The proton solvents such as methanol, ethanol are used in 10 to 50 times the amount of the compound (VIII) or (IX). The mixed solvents with chloroform or the like may be used to enhance the solubility. The purification of the reaction product can be performed by a silica gel chromatography.

Subsequently, the arylsulfonyl group at 23 position is removed reductively from the compound (X) or (XI) using e.g., 5% Na-Hg in 2 to 20 times the amount of said compound to afford a compound of formula (XII) or (XIII). This reaction is carried out at a temperature of 0 to 50°C, usually room temperature for 5 to 50 hours in proton solvents such as methanol, ethanol, etc. which are used in 5 to 50 times the amount of

said compound. The solvents may contain disodium hydrogenphosphate. Purification of the reaction product can be performed by a silica gel chromatography.

The protecting group (PTAD) for 5,7-diene is removed from the compound (XII) or (XIII) using suitable reducing agents such as $LiAlH_4$, DIBAL (diisobutyl aluminum hydride), etc., to afford the 5,7-diene compound of formula (XIV) or (XV). This reaction is carried out at a temperature between room temperature and boiling point of the solvent for 0.5 to 5 hours in suitable solvents such as THF, ether, etc. The reducing agents are used in 1 to 10 times the amount of the compound (XII) or (XIII) and the solvents are used in 5 to 50 times that of said compound. Purification of the reaction product can be performed by crystallization from ethanol, methanol, acetone or the like.

Subsequently, the 5,7-diene compound of formula (XIV) or (XV) is subjected to irradiation followed by thermal isomerization to form the vitamin D derivative of formula (XVI) or (XVII). The photoreaction is performed at a temperature of 0 to 50 °C in suitable solvents such as ethanol, THF, ether, toluene which are used in 5 to 50 times the amount of said compound, preferably in a gas stream such as nitrogen or argon. The thermal isomerization is performed at a temperature between room temperature and boiling point of the solvent in suitable solvents such as ethanol, methanol, toluene or the like, preferably in a gas stream such as nitrogen or argon.

The vitamin D derivative of formula (XVI) or (XVII) is then tosylated and the tosylate is displaced by solvolysis, e.g., methanol treatment under a basic condition (e.g., $NaHCO_3$) to give the cyclovitamin D compound of formula (XVIII) or (XIX). The tosylation is carried out under a similar condition to that of compound (IV). The cyclization is carried out at a temperature between room temperature and boiling point of methanol for 1 to 10 hours. The base, e.g., $NaHCO_3$ is used in 1 to 50 times the amount of the tosylate and methanol is used in 10 to 150 times the amount of the tosylate. Purification of the reaction product can be performed by a silica gel chromatography.

The cyclovitamin D compound of formula (XVIII) or (XIX) is subjected to allylic oxidation (e.g., using $SeO_2$/t-BuOOH) to form the 1$\alpha$-hydroxy-3,5-cyclovitamin D derivative of formula (XX) or (XXI). This reaction is carried out at a temperature of 0 °C to room temperature for 0.5 to 5 hours in suitable solvents such as dichloromethane, dichloroethane, THF, etc. For instance, $SeO_2$ is used in 0.1 to 5 times the amount of the compound (XVIII) or (XIX) and t-BuOOH is used in 1 to 10 times the amount of said compound. Purification of the reaction product can be performed by a silica gel chromatography.

The 1$\alpha$-hydroxy-3,5-cyclovitamin D derivative of formula (XX) or (XXI) is solvolyzed with e.g., acetic acid to afford the 3$\beta$-acetyl compound of formula (XXII) or (XXIII) which is then subjected to saponification to give 1$\alpha$-hydroxy vitamin $D_7$ of formula (I) or 1$\alpha$-hydroxy vitamin $D_4$ of formula (II). The solvolyzing is performed at a temperature of 30 to 100 °C for 0.1 to 1 hour. For instance, acetic acid is used in 1 to 50 times the amount of the compound (XX) or (XXI). The 3$\beta$-acetate compound is purified by a silica gel chromatography. The saponification is performed at a temperature of 0 to 50 °C in suitable solvents such as methanol, ethanol, using an alkali such as NaOH, KOH, etc. Purification of the product is conducted by a silica gel chromatography followed by crystallization from suitable solvents such as ether, acetone, hexane or the mixed solvents.

The sulfone derivative of formula (VI) or (VII) can be readily prepared, as shown in Process Scheme II, by treating the sulfone derivative of formula (XXIV) or (XXV) with phosphorus oxychloride or methanesulfonyl chloride under a basic condition to form the olefin derivative of formula (XXVI) or (XXVII) and then subjecting the olefin derivative to catalytic hydrogenation.

1$\alpha$-Hydroxy vitamin $D_7$ and 1$\alpha$-hydroxy vitamin $D_4$ of the present invention possess reduced side effects as well as more improved activity to increase a mineral bone density and more improved activity to induce a cell differentiation as compared with known active forms of vitamin D. Therefore, the present compounds of formulae (I) and (II) can be used to treat or prevent vitamin D deficiency diseases and related diseases, especially osteoporosis and psoriasis.

Thus the present invention further provides pharmaceutical compositions which comprise as active ingredients 1$\alpha$-hydroxy vitamin $D_7$ and/or 1$\alpha$-hydroxy vitamin $D_4$, which have reduced side effects and lower toxicity as compared with the known active forms of vitamin $D_3$.

The pharmaceutical compositions of the present invention may contain other therapeutically effective ingredients such as calcium salts, e.g., calcium lactate, calcium phosphate, calcium gluconate and calcium hydrogenphosphate and/or other trace element ingredients such as salts of magnesium, manganese, iron, copper, zinc and iodine and/or other vitamins such as vitamin A, vitamin $B_1$ and vitamin $B_2$, nicotinic acid amide, pantothenic acid or its salts (e.g., calcium salt), vitamin $B_6$, vitamin $B_{12}$, folic acid, vitamin C and vitamin E or the like.

The pharmaceutical compositions comprising 1$\alpha$-hydroxy vitamin $D_7$ and 1$\alpha$-hydroxy vitamin $D_4$ as active ingredients can be formulated into solid and liquid preparations according to conventional methods of

pharmacy.

The solid preparations can be orally administered, including tablets, coated tablets, capsules, dragees, powders, granules or the like. In the formulation of solid preparations, the active ingredients can be employed in admixture with antioxidants and pharmaceutically acceptable vehicles or solid carriers. Antioxidants include ascorbic acid, butylated hydroxyanisole or hydroquinone, etc. Suitably pharmaceutically acceptable vehicles or solid carriers include binders such as gelatine, sorbitol, tragacanth gum, gum arabic, CMC and polyvinyl pyrrolidone; fillers such as lactose, sugar, corn starch, calcium phosphate and silica; lubricants such as magnesium stearate, talc and polyethyleneglycol; disintegrating agents such as potato starch and wetting agents such as sodium lauryl sulfate, glycerol monostearate and the like. The solid preparations are preferably formulated in unit dosage form, each unit containing 0.01 to 50 $\mu$g, preferably 0.05 to 10 $\mu$g of 1$\alpha$-hydroxy vitamin $D_7$ or 1$\alpha$-hydroxy vitamin $D_4$.

The liquid preparations can be administered parenterally including injections, solutions, suspensions, emulsions or the like. In the formulation of liquid preparations, the active ingredients are dissolved in edible or absorbable oily materials. The oily materials include vegetable oils such as soybean oil, rape oil, corn oil, peanut oil, almond oil, coconut oil, cacao butter; animal oils such as tallow oils and fish liver oils; synthetic triglycerides such as middle chain long fatty acid triglycerides and oily ester materials such as glycerol monostearate, glycerol monooleate, glycerol dioleate, glycerol monolaurate, glycerol dilaurate, polysorbate 80. The liquid preparations can be admixed with various additives, vehicles or the like which are employed in the formulation of solid preparations. Advantageously, antioxidants such as ascorbic acid, butylated hydroxyanisole or hydroquinone are incorporated into liquid preparations to increase the storage life of the active ingredients.

The liquid preparations can be administered as such or in the form encapsulated in soft capsule. They can be formulated in unit dosage form such as soft capsule, each unit containing 0.01 to 50 $\mu$g, preferably 0.05 to 10 $\mu$g of 1$\alpha$-hydroxy vitamin $D_7$ or 1$\alpha$-hydroxy vitamin $D_4$.

The dosage of the compounds according to the present invention is generally about 0.1 to 30 $\mu$g/day for the treatment of adult, depending on the age, body weight, general state of health, sex, mode of administration or the like.

The invention is further illustrated by the following non-limitative examples.

Example 1

Synthesis of 1$\alpha$-hydroxy vitamin $D_7$ (I)

(1) (22E)-5$\alpha$,8$\alpha$-(4-Phenyl-1,2-urazolo)-6,22-ergostadien-3$\beta$-ol 3$\beta$-t-butyldimethylsilyl ether (III)

Ergosterol (102 g) and 4-phenyl-1,2,4-triazoline-3,5-dione (42 g) were added to one liter of methylene chloride and the mixture was allowed to react. The reaction solution was concentrated and the residue was crystallized from acetone to afford 87.9 g of 5$\alpha$,8$\alpha$-(4-phenyl-1,2-urazolo)-6-ergosten-3$\beta$-ol. This was dissolved in DMF (400 ml), to which was added imidazole (27.2 g) and t-butyldimethylchlorosilane (27.2 g) and the mixture was stirred for 2 hrs. The reaction solution was poured into ice-cold water and extracted with chloroform. The chloroform layer was washed with water, condensed and crystallized from ethanol to give 102 g of the title compound. m.p. 190 °C

NMR $\delta$($CDCl_3$)、0.06(3H, s)、0.11(3H, s)、0.88(9H, s)、0.96(3H, s)、1.0 2(3H, d, J = 6.8Hz)、4.40(1H, m)、5.20(2H, m)、6.07 and 6.20(2H, ABq, J = 8 .1Hz)、7.30-7.45(5H, m)

(2) 5$\alpha$,8$\alpha$-(4-Phenyl-1,2-urazolo)-23,24-dinor-6-cholene-3$\beta$,22-diol 3$\beta$-t-butyldimethylsilyl ether (IV)

(22E)-5$\alpha$,8$\alpha$-(4-Phenyl-1,2-urazolo)-6,22-ergostadien-3$\beta$-ol 3$\beta$-t-butyldimethylsilyl ether (III) (102 g) was dissolved in one liter of methylene chloride containing 1% pyridine and the solution was cooled below -65 °C. Ozone was passed through the solution at a rate of 1.5 g/hr for 5 hrs, and thereafter sodium boron hydride (15 g) and methanol (100 ml) were added and raised to 0 °C. To the reaction solution was added diluted hydrochloric acid and stirred. The methylene chloride layer was separated, washed with sodium hydrogencarbonate, dried and the solvent was distilled away. Purification of the residue by a silica gel column gave 43 g of the recovered starting compound (III) and 44.3 g of the title compound. m.p. 173 °C

NMR $\delta$($CDCl_3$)、0.08(3H, s)、0.10(3H, s)、0.82(3H, s)、0.88(9H, s)、0.9 7(3H, s)、1.07(3H, d, J = 6.8Hz)、4.40(1H, m)、6.13 and 6.29(2H, ABq, J = 8 3HZ)、7.29-7.46(5H, m)

(3) 22-Iodo-5α,8α-(4-phenyl-1,2-urazolo)-23,24-dinor-6-cholen-3β-ol 3β-t-butyldimethylsilyl ether (V)

5α,8α-(4-Phenyl-1,2-urazolo)-23,24-dinor-6-cholene-3β,22-diol 3β-t-butyldimethylsilyl ether (IV) (44.3 g) was dissolved in pyridine (500 ml), p-toluenesulfonyl chloride (27 g) was added and the solution was reacted under ice-cooling for 17 hrs. The reaction solution was poured into ice-cold water, stirred for 0.5 hr, extracted with ethyl acetate, washed with successive, diluted hydrochloric acid, aqueous sodium hydrogencarbonate solution and saturated saline water and dried. The solvent was distilled away to afford the crude 22-tosylate (43.4 g). To a solution of the crude 22-tosylate (36.2 g) in acetone (400 ml) was added sodium iodide (36 g) and the solution was refluxed for 4 hrs. The solvent was distilled away, the residue to which water was added, was extracted with chloroform, washed with water, dried and the solvent was distilled away. Crystallization of the residue from hexane gave 26.1 g of the title compound. m.p. 168 °C

NMR δ($CDCl_3$)、0.08(3H, s)、0.11(3H, s)、0.84(3H, s)、0.88(9H, s)、0.9 6(3H, s)、1.05(3H, d, J = 6.4Hz)、3.09-3.17(2H, m)、3.32(1H, dd, J = 2.4 and 9.3Hz)、4.40(1H, m)、6.21 and 6.36(2H, ABq, J = 8.3Hz)、7.29-7.46(5H , m)

(4) (24R)-5α,8α-(4-Phenyl-1,2-urazolo)-6-ergosten-3β-ol (XII)

(3S)-2,3-Dimethylbutylphenyl sulfone (VI) (7.4 g) was dissolved in dry THF (50 ml) and cooled to -20 °C, then 1.65 N n-butyl lithium (20 ml) was added dropwise and the mixture was stirred for one hour. 1,3-Dimethyl-2-imidazolidinone (15 ml) was added, then a solution of 22-iodo-5α,8α-(4-phenyl-1,2-urazolo)-23,24-dinor-6-cholen-3β-ol 3-tert-butyldimethylsilyl ether (V) (18.2 g) in dry THF was added dropwise and the mixture was reacted at room temperature for 2 hrs. The reaction solution was extracted with a saturated aqueous ammonium chloride solution and ethyl acetate, the organic layer was washed with a saturated sodium chloride solution and dried. Removal of the solvent by distillation gave 24.8 g of a crude product, (24S)-23-phenylsulfonyl-5α,8α-(4-phenyl-1,2-urazolo)-6-ergosten-3β-ol-tert-butyldimethylsilyl ether (VIII). This crude product was dissolved in 5:3 methanol/chloroform (800 ml), p-toluene solfonic acid monohydrate (0.5 g) was added and the mixture was stirred at room temperature for one hour. Potassium carbonate was added, a supernatant was concentrated and water was added. The mixture was extracted with ethyl acetate and the solvent was distilled off. Purification of the residue by a silica gel column gave 18.7 g of (24S)-23-phenylsulfonyl-5α,8α-(4-phenyl-1,2-urazolo)-6-ergosten-3β-ol (X). The 23-phenylsulfonyl (18.7 g) was dissolved in methanol (500 ml), disodium hydrogenphosphate (10 g) and 5% sodium amalgam (100 g) were added and the mixture was stirred. After 17 hours, the liberated mercury was removed, the methanol solution was concentrated and extracted with water and chloroform, and the chloroform layer was concentrated. Purification of the residue by a silica gel column afforded 9.5 g of the title compound (XII).

NMR δ($CDCl_3$) : 0.76-0.81(9H, m)、0.84(3H, d, J = 6.9Hz)、0.93(3H, d, J = 6.8Hz)、0.96(3H, s)、2.33(1H, m)、2.50(1H, m)、3.16(1H, dd, J = 13.6 and 3.9Hz)、4.44 (1H, m)、6.23 and 6.40(2H, ABq, J = 8.3Hz)、7.30-7.46(5H, m)

(5) (24R)-Ergosta-5,7-diene-3β-ol (XIV)

To a suspension of lithium aluminum hydride (2 g) in dry THF (120 ml) was added dropwise a mixture of (24R)-5α,8α-(4-phenyl-1,2-urazolo)-6-ergosten-3β-ol (XII) (9.5 g) and dry THF (120 ml) and the mixture was reacted under reflux for 2 hrs. After decomposition of excessive lithium aluminum hydride in the usual way, the THF layer was separated and concentrated. Recrystallization of the residue from ethanol afforded 5.7 g of the title compound (XIV).

m.p. 165 °

NMR δ($CDCl_3$) : 0.62(3H, s)、0.78(3H, d, J = 6.8Hz)、0.80(3H, d, J = 6.8Hz)、0.85(3H, d, J = 6.9Hz)、0.93(3H, d, J = 6.9Hz)、0.96(3H, s)、3.64(1H, m)、5.39(1H, m)、5.57(1H, m)

| Elemental analysis for $C_{28}H_{46}O$ | | |
|---|---|---|
| Found | C 84.40% | H 11.69% |
| Calc'd | C 84.36% | H 11.63% |

(6) Vitamin $D_7$ (XVI)

A solution of (24R)-ergosta-5,7-diene-3β-ol (XIV) (5g) in THF (1 lit.) was irradiated for one hour with high pressure mercury lamp (Usio UM-452, Usio Denki Co., Ltd.) using 1.5% potassium nitrate as a filter under water-cooling in an argon gas stream. The reaction solution was concentrated and previtamin $D_7$ (1.5 g) and the 5,7-diene (2 g) were separated on a silica gel column. The separated 5,7-diene (2 g) was irradiated with light in a similar manner. The previtamin $D_7$ was formed into the ethanol solution which was then refluxed in a nitrogen stream for 2 hrs to distil off the solvent. Purification of the residue by a silica gel column gave 1.65 g of the title compound.

NMR δ($CDCl_3$) : 0.54(3H, s)、0.77(3H, d, J=6.3Hz)、0.80(3H, d, J=6.9Hz)、0.85(3H, d, J=6.9Hz)、0.91(3H, d, J=5.9Hz)、2. 57(1H, dd, J=12.7Hz and 3.5Hz)、2.82(1H, m)、3.95(1H, m)、4.82(1H, d, J=2.5Hz)、5.05(1H, s)、6 .03 and 6.23(2H, ABq, J=11.2Hz)

(7) 1-Hydroxy-3,5-cyclovitamin $D_7$ (XX)

To a solution of vitamin $D_7$ (XVI) (1.65 g) in pyridine (50 ml) was added p-toluenesulfonyl chloride (5.0 g) and the mixture was stirred for 17 hrs. The reaction solution was cooled, water was added and the reaction mixture was treated in the usual way to afford the crude tosylate (2.0 g). To a solution of the crude tosylate (2.0 g) in methanol (100 ml) was added sodium hydrogencarbonate (9 g) and the mixture was reacted under reflux for 5 hrs. Methanol was distilled off, water was added, the reaction solution was extracted with ethyl acetate and the solvent was distilled off. Purification of the residue by a silica gel column gave 1.44 g of 3,5-cyclovitamin $D_7$ (XVIII). To methylene chloride (45 ml) was added selenium dioxide (0.28 g) and a 2,2,4-trimethylpentane solution (2.7 ml) of 3.0 M tert-butyl hydroperoxide at room temperature, which was stirred for one hour at the same temperature. To the stirred mixture was added at 5°C a methylene chloride solution of the cyclo compound (XVIII) (1.44 g), the mixture was stirred for one hour and 10% aqueous sodium hydroxide solution (50 ml) was added to cease the reaction. The methylene chloride layer was separated, dried and concentrated. Purification of the residue by a silica gel column gave 0.62 g of the title compound.

NMR δ($CDCl_3$) : 0.53(3H, s)、0.77(3H, d, J=6.3Hz)、0.80(3H, d, J=6.9Hz)、0.85(3H, d, J=6.9Hz)、0.91(3H, d, J=6.4Hz)、2.26(1H, m)、2.64 (1H, m)、3.26(3H, s)、4.19(1H, d, J=9.2Hz)、4.21(1H, br)、4.94-(1H, d, J=9.3Hz)、5 .17(1H, s)、5.24(1H, s)

(8) 1α-Hydroxy vitamin $D_7$ 3β-acetate (XXII)

A solution of 1-hydroxy-3,5-cyclovitamin $D_7$ (XX) (0.62 g) in acetic acid (10 ml) was reacted at 55°C for 15 minutes. The reaction solution was poured into an aqueous sodium hydrogencarbonate solution and extracted with ethyl acetate and the solvent was distilled off. Purification of the residue by a silica gel column gave 0.27 g of the title compound. This column purification could separate 1β-hydroxy vitamin $D_7$ 3β-acetate (50 mg) in the first effluent and 1α-hydroxy-5,6-transvitamin $D_7$ 3β-acetate (100 mg) in the third effluent.

1α-Hydroxy vitamin $D_7$ 3β-acetate

NMR δ($CDCl_3$) : 0.54(3H, s)、0.77(3H, d, J=6.3Hz)、0.80(3H, d, J=6.9Hz)、0.85(3H, d, J=6.9Hz)、0.91(3H, d, J=5.9Hz)、2.04(3H, s)、2.40(1H, dd, J=13.6Hz and 6.3Hz)、2.59(1H, dd, J=13.7Hz and 3.4Hz)、2.81 (1H, dd, J= 12.2Hz and 2.9Hz)、4.41(1H, br)、5.02(1H, s)、5.19(1H, m)、5.34(1H, s)、6.02 and 6.34(2H, ABq, J=11.3Hz)

1β-Hydroxy vitamin $D_7$ 3β-acetate

NMR δ($CDCl_3$) : 0.53(3H, s)、0.77(3H, d, J=6.3Hz)、0.80(3H, d, J=6.9Hz)、0.85(3H, d, J=6.9Hz)、0.91(3H, d, J=6.3Hz)、2.06(3H, s)、2.60(1H, dd, J=12.7Hz and 3.9Hz)、2.82(1H, m)、4.18(1H, br)、4.98-(1H, m)、5.01(1H, s)、5.36(1H, s)、6.00 and 6.37(2H, ABq, J=11.2Hz)

1α-Hydroxy-5,6-transvitamin $D_7$ 3β-acetate

NMR δ($CDCl_3$) : 0.55(3H, s)、0.78(3H, d, J=6.3Hz)、0.81(3H, d, J=6.8Hz)、0.86(3H, d, J=6.3Hz)、0.92(3H, d, J=5.8Hz)、2.04(3H, s)、2.47(1H, dd, J=14.7Hz and 6.9Hz)、2.59(1H, dd, J=11.7Hz and 3.9Hz)、2.85(1H, m)、4. 49(1H, br)、4.99(1H, s)、5.13(1H, s)、5.25(1H, m)、5.81 and 6.57(2H, AB q, J=11.2Hz)

(9) 1α-Hydroxy vitamin $D_7$ (I)

To 1α-hydroxy vitamin $D_7$ 3β-acetate (XXII) (0.27 g), 10% ethanolic KOH (5ml) was added and the mixture was stirred for 0.5 hr. The reaction solution was poured into water, extracted with ethyl acetate and the solvent was distilled off. Purification of the residue by a silica gel column afforded 0.22 g of the title compound. m.p. 174 °C (ether-hexane)

$[\alpha]_D^{25°}$ +60.1 ° (c = 0.1、EtOH)

NMR δ($CDCl_3$) : 0.54(3H, s)、0.77(3H, d, J=6.3Hz)、0.80(3H, d, J=6.3Hz)、0.85(3H, d, J=6.9Hz)、0.91(3H, d, J=6.4Hz)、2.31(1H, dd, J=13.2Hz and 6.3Hz)、2.59(1H, dd, J=13.7Hz and 3.4Hz)、2.81(1H, dd, J=11.3Hz and 3.0Hz)、4.23(1H, m)、4.43(1H, m)、5.01(1H, s)、5.33(1H, s)、6.02 and 6. 348(2H, ABq, J=11.2Hz)

MS m/z (relative strength, %) 415($M^+$+1, 31), 414($M^+$, 100), 396(52), 378(27), 296(12), 287(38), 269(26), 251(24), 174(55)

Example 2

Synthesis of 1α-hydroxy vitamin $D_4$ (II)

(1) (24S)-5α,8α-(4-phenyl-1,2-urazolo)-6-ergosten-3β-ol (XIII)

(3R)-2,3-Dimethylbutylphenyl sulfone (VII) (13.0 g) was dissolved in dry THF (50 ml) and cooled to -20 °C, then 1.65 N n-butyl lithium (25 ml) was added dropwise and the mixture was stirred for one hour. 1,3-Dimethyl-2-imidazolidinone (22 ml) was added, then a solution of 22-iodo-5α,8α-(4-phenyl-1,2-urazolo)-23,24-dinor-6-cholen-3β-ol 3-tert-butyldimethylsilyl ether (V) (28.0 g) in dry THF was added dropwise and the mixture was reacted at room temperature for 2 hrs. The reaction solution was extracted with a saturated aqueous ammonium chloride solution and ethyl acetate, the organic layer was washed with a saturated sodium chloride solution and dried. Removal of the solvent by distillation gave 26.7 g of a crude product, (24R)-23-phenylsulfonyl-5α,8α-(4-phenyl-1,2-urazolo-6-ergosten-3β-ol t-butyldimethylsilyl ether (IX). This crude product was dissolved in 9:1 methanol/chloroform (1000 ml), p-toluene sulfonic acid monohydrate (1.0 g) was added and the mixture was stirred at room temperature for one hour. Potassium carbonate was added, methanol was concentrated, water was added, and the solution was extracted with ethyl acetate and the solvent was distilled off. Purification of the residue by a silica gel column gave 24.3 g of (24R)-23-phenylsulfonyl-5α,8α-(4-phenyl-1,2-urazolo)-6-ergosten-3β-ol (XI). The 23-phenylsulfonyl (24.3 g) was dissolved in methanol (500 ml), disodium hydrogenphosphate (12.0 g) and 5% sodium amalgam (100 g) were added and the mixture was stirred at room temperature. After 17 hours, the liberated mercury was removed, the methanol solution was concentrated and extracted with water and chloroform, and the chloroform layer was concentrated. Purification of the residue by a silica gel column afforded 7.63 g of the title compound (XIII).

NMR δ($CDCl_3$) : 0.76-0.82(9H, m)、0.85(3H, d, J=6.8Hz)、0.94(3H, d, J= 6.4Hz)、0.97(3H, s)、2.34(1H, m)、2.51(1H, m)、3.17(1H, dd, J=13.7 and 4.9Hz)、4.46(1H, m)、6.24 and 6.41(2H, ABq, J=8.5Hz)、7.30-7.45(5H,m)

(2) (24S)-Ergosta-5,7-diene-3β-ol (XV)

To a suspension of lithium aluminum hydride (1.6 g) in dry THF (100 ml) was added dropwise a mixture of (24S)-5α,8α-(4-phenyl-1,2-urazolo)-6-ergosten -3β-ol (XIII) (7.63 g) and dry THF (100 ml) and the mixture was reacted under reflux for 2 hrs. After decomposition of excessive lithium aluminum hydride in the usual way, the THF layer was separated and concentrated. Recrystallization of the residue from ethanol afforded 3.74 g of the title compound (XV).

m.p. 145 °C

NMR δ($CDCl_3$) : 0.62(3H, s)、0.78(3H, d, J=6.6Hz)、0.79(3H, d, J=6.8Hz)、0.86(3H, d, J=6.8Hz)、0.93-0.96(6H, m)、3.63(1H, m)、5.38(1H, m)、5.5 7(1H, m)

(3) Vitamin $D_4$ (XVII)

A solution of (24S)-ergosta-5,7-diene-3β-ol (XV) (3.74 g) in THF (1 lit.) was irradiated for one hour with a high pressure mercury lamp (Usio UM-452, Usio Denki Co., Ltd.) using 1.5% potassium nitrate as a filter under water-cooling in an argon gas stream. The reaction solution was concentrated, and previtamin $D_4$ -

(0.98 g) and the 5,7-diene (0.78 g) were separated on a silica gel column. The separated 5,7-diene (0.78 g) was irradiated with light in a similar manner. The previtamin $D_4$ was formed into the ethanol solution which was then refluxed in a nitrogen stream for 2 hrs and the solvent was distilled away. Purification of the residue by a silica gel column gave 0.62 g of the title compound.

NMR δ($CDCl_3$) : 0.54(3H, s)、0.77(3H, d, J=6.6Hz)、0.78(3H, d, J=6.8Hz)、0.86(3H, d, J=6.8Hz)、0.92(3H, d, J=6.1Hz)、2.58(1H, m)、2.83(1H, m)、3.95(1H, m)、4.82(1H, m)、5.05(1H, m)、6.03 and 6.24-(2H, ABq, J=11.2Hz)

(4) 1α-Hydroxy vitamin $D_4$ 3β-acetate (XXIII)

To a solution of vitamin $D_4$ (XVII) (0.62 g) in pyridine (10 ml) was added p-toluenesulfonyl chloride (2.0 g) and the mixture was stirred for 17 hrs. The reaction solution was cooled, water was added and the reaction mixture was treated in the usual way to afford the crude tosylate (0.9 g). To a solution of the crude tosylate (0.9 g) in methanol (100 ml) was added sodium hydrogencarbonate (3 g) and the mixture was reacted under reflux for 5 hrs. Methanol was distilled off, water was added, the reaction solution was extracted wtih ethyl acetate and the solvent was distilled off. Purification of the residue by a silica gel column gave 0.64 g of 3,5-cyclovitamin $D_4$ (XIX). To methylene chloride (30 ml) was added selenium dioxide (0.18 g) and 2,2,4-trimethylpentane solution (1.8 ml) of 3.0 M tert-butyl hydroperoxide at room temperature, which was stirred for one hour. To the mixture was added at 5°C a methylene chloride solution of the cyclovitamin D (XIX) (0.64 g), the mixture was stirred for 0.75 hour and 10% aqueous sodium hydroxide solution (50 ml) was added to cease the reaction. The methylene chloride layer was separated and concentrated. The residue was purified by a silica gel column to afford 0.38 g of 1-hydroxy-3,5-cyclovitamin $D_4$ (XXI). An acetic acid solution (10 ml) of the compound (XXI) (0.38 g) was reacted at 55°C for 15 minutes. The reaction solution was poured into an aqueous sodium hydrogencarbonate solution and extracted with ethyl acetate. The solvent was distilled away. Purification of the residue by a silica gel column gave 0.16 g of the title compound. This column purification could separate 1β-hydroxy vitamin $D_4$ 3β-acetate (30 mg) in the first effluent and 1α-hydroxy-5,6-transvitamin $D_4$ 3β-acetate (80 mg) in the third effluent.

1α-Hydroxy vitamin $D_4$ 3β-acetate (XXIII)

NMR δ($CDCl_3$) : 0.54(3H, s)、0.78(3H, d, J=6.4Hz)、0.79(3H, d, J=6.8Hz)、0.86(3H, d, J=6.8Hz)、0.93(3H, d, J=6.4Hz)、2.41(1H, dd, J=13.7Hz and 6. 4Hz)、2.59(1H, dd, J=14.2Hz and 3.4Hz)、2.81(1H, m)、4.40(1H, m)、5 .02(1H, s)、5.21(1H, s)、5.35(1H, s)、6. 03 and 6.34(2H, ABq, J=11.3Hz)

1β-Hydroxy vitamin $D_4$ 3β-acetate

NMR δ($CDCl_3$) : 0.53(3H, s)、0.78(3H, d, J=6.8Hz)、0.79(3H, d, J=6.8Hz)、0.86(3H, d, J=6.8Hz)、0.92(3H, d, J=5.9Hz)、2.61(1H, m)、2.81(1H, m)、4.17(1H, m)、4.98(1H, m)、5.01(1H, s)、5.36(1H, s)、6.00 and 6.38(2H, A Bq, J=11.2Hz)

1α-Hydroxy-5,6-transvitamin $D_4$ 3β-acetate

NMR δ($CDCl_3$) : 0.54(3H, s)、0.78(3H, d, J=6.8Hz)、0.79(3H, d, J=6.8Hz)、0.86(3H, d, J=6.8Hz)、0.93(3H, d, J=5.9Hz)、2.49(1H, dd, J=14.2Hz and 7.3Hz)、2.74(1H, m)、2.85(1H, m)、4.48(1H, m)、4.99-(1H, s)、5.13(1H, s)、5.25(1H, m)、5.81 and 6.57(2H, ABq, J=11.2Hz)

(5) 1α-Hydroxy vitamin $D_4$ (II)

To 1α-hydroxy vitamin $D_4$ 3β-acetate (XXIII) (0.16 g), 10% ethanolic KOH (5 ml) was added and the mixture was stirred for 0.5 hr. The reaction solution was poured into water, extracted with ethyl acetate and the solvent was distilled off. Purification of the residue by a silica gel column afforded 0.09 g of the title compound.

m.p. 148-149°C (ether-hexane)

$[\alpha]_D^{25°}$ +56.0 (C=0.25、EtOH)

NMR δ($CDCl_3$) : 0.54(3H, s)、0.78(3H, d, J=6.8Hz)、0.79(3H, d, J=6.8Hz)、0.86(3H, d, J=6.8Hz)、0.92(3H, d, J=6.4Hz)、2.32(1H, dd, J=13.4Hz and 6.5Hz)、2.66(1H, m)、2.83(1H, m)、4.23(1H, m)、4.43-(1H, m)、5.01(1H, s )、5.33(1H, s)、6.02 and 6.38(2H, ABq, J=11.2Hz)

MS m/z (relative strength, %) 415($M^+$+1, 30), 414($M^+$, 100), 396(61), 378(37), 326(16), 287(48), 269(43), 251(31), 174(62)

Example 3

Activity test of 1α-hydroxy vitamin $D_4$ and 1α-hydroxy vitamin $D_7$

Female Wistar rats (9 week-old) were given a usual feed containing 1.17% calcium throughout the entire experiment. Seventy two rats were subjected to bilateral ovariectomy and right sciatic nerve amputation. Rats were divided into 9 groups of eight animals each. Another group of eight rats was subjected to sham operation. Over a period of 6 weeks after operation, each group was orally administered either 1α-hdyroxy vitamin $D_3$, 1α-hydroxy vitamin $D_7$ or 1α-hydroxy vitamin $D_4$ dissolved in 0.5% ethanol/porpyleneglycol (vehicle) at the frequency of 5 days per week. The sham operated group and control group were orally administered the vehicle alone. The next day after final administration, rats were killed, blood was drawn from abdominal cave and right femur was extracted. The bone volume of femur was measured in accordance with Archimedes' principle. After heat treatment at 700 °C for 24 hrs, the weight of bone ash was measured. From these measured values, the bone density (ash weight/volume) of each femur was calculated. Serum calcium concentration was determined according to usual method. The results are shown in Table 1.

Table 1

| Test group | Dosage (μg/kg/day) | Number of rats | Bone density ($mg/mm^3$) | Serum calcium concentration (mg/dl) |
|---|---|---|---|---|
| Sham | | 8 | 0.651±0.009 | 10.8±0.2 |
| Control | | 8 | 0.539±0.003 | 10.4±0.1 |
| Administration | | | | |
| 1α-OH-$D_3$ | 0.02 | 8 | 0.556±0.005 | 10.8±0.1 |
| | 0.1 | 8 | 0.545±0.007 | 11.0±0.1 |
| | 0.5 | 8 | 0.560±0.006 | 12.4±0.2 |
| 1α-OH-$D_7$ | 0.5 | 8 | 0.581±0.004 | 10.5±0.1 |
| | 2.5 | 8 | 0.585±0.007 | 10.7±0.3 |
| | 12.5 | 8 | 0.589±0.004 | 10.9±0.5 |
| 1α-OH-$D_4$ | 0.5 | 8 | 0.576±0.006 | 10.5±0.2 |
| | 2.5 | 8 | 0.583+0.003 | 11.0±0.4 |

The data of Table 1 indicate that 1α-hydroxy vitamin $D_7$ and 1α-hydroxy vitamin $D_4$ exhibit much greater increase in bone density, i.e., potent activity to increase a bone mineral density and lesser increase in serum calcium concentration than known 1α-hydroxy vitamin $D_3$.

Example 4

Orally administrable 1α-hydroxy vitamin $D_7$ composition

1α-hydroxy vitamin $D_7$ (10 mg) was added to one liter of middle chain fatty acid triglyceride (MCT) and stirred to form a uniform solution with 10 μg/ml of 1α-hydroxy vitamin $D_7$ concentration. 1/5 ml portion of the resultant solution was capsuled in gelatin by a conventional technique. In a similar manner, one capsule

was prepared from each solution with 20 μg/ml of 1α-hydroxy vitamin $D_7$ concentration.

**Claims**

1. A 1α-hydroxy vitamin D compound of formula (I) or (II)

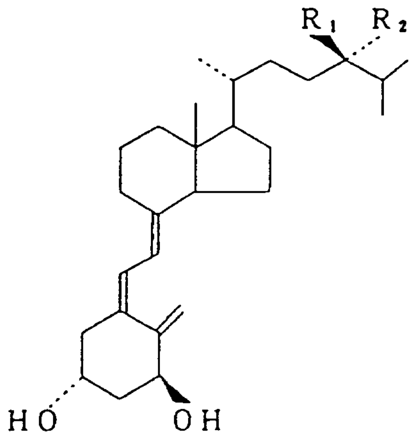

(I) : $R_1 = CH_3$, $R_2 = H$

(II) : $R_1 = H$, $R_2 = CH_3$

2. A compound of claim 1 wherein said compound is 1α-hydroxy vitamin $D_7$.

3. A compound of claim 1 wherein said compound is 1α-hydroxy vitamin $D_4$.

4. A pharmaceutical composition for preventing or treating vitamin D deficient diseases, which comprises as an active ingredient an effective amount of a compound of formula (I) or (II)

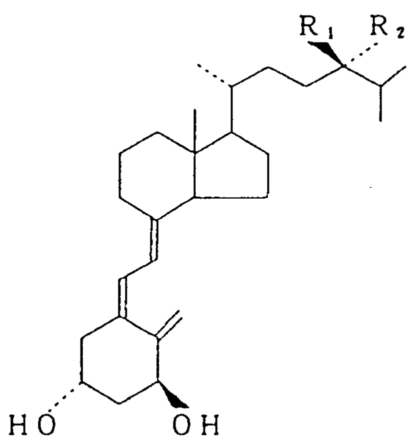

(I) : $R_1 = CH_3$, $R_2 = H$

(II) : $R_1 = H$, $R_2 = CH_3$

in combination with a pharmaceutically acceptable vehicle.

5. A pharmaceutical composition for preventing or treating osteoporosis, which comprises a physiologically acceptable vehicle and an effective amount of a compound of formula (I) or (II)

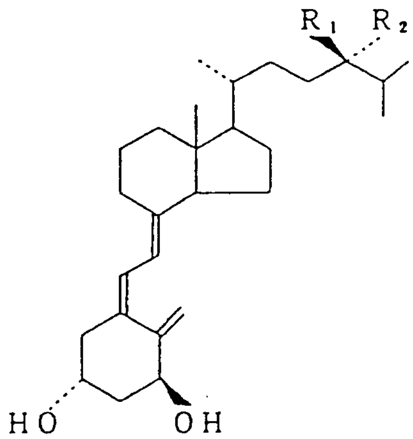

(I) : $R_1=CH_3$, $R_2=H$

(II) : $R_1=H$, $R_2=CH_3$

**6.** Use of a compound of formula (I) or (II)

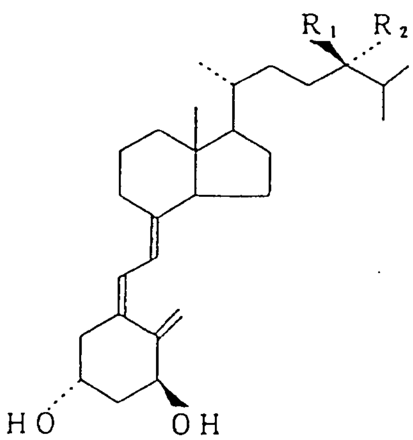

(I) : $R_1=CH_3$, $R_2=H$

(II) : $R_1=H$, $R_2=CH_3$

for the preparation of medicaments for treating osteoporosis.

**7.** A method of preparing a 1α-hydroxy vitamin $D_7$ compound of formula (I)

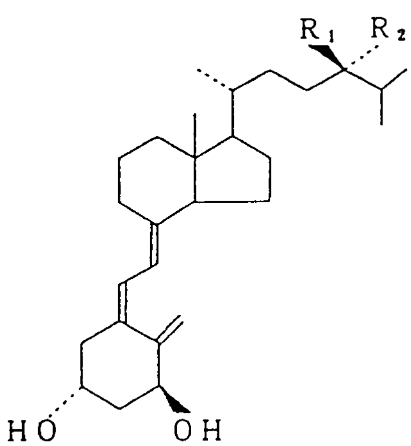

(I) : $R_1=CH_3$, $R_2=H$

,

which comprises:

(a) condensing the 22-halo compound of formula (V)

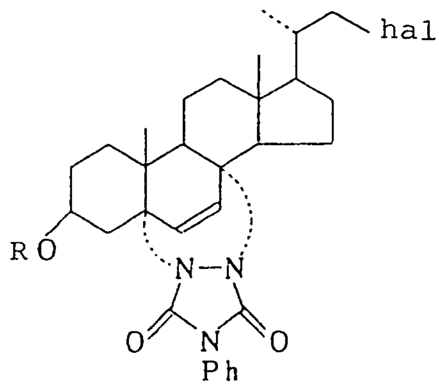

(V)

with a sulfone derivative of formula (VI)

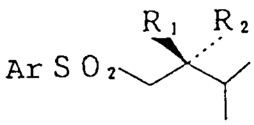

(VI) : $R_1$=Me, $R_2$=H

to form a compound of formula (VIII)

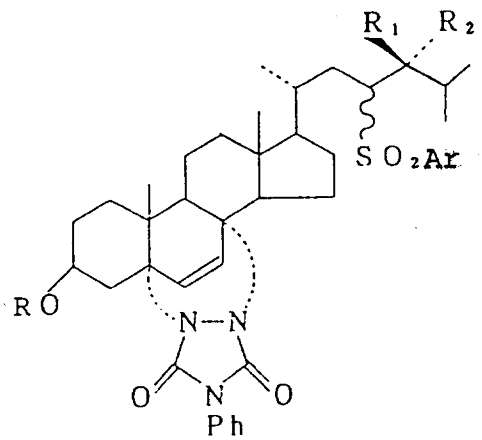

(VIII) : $R_1$=Me, $R_2$=H

;

(b) removing from the resultant compound (VIII) the protecting group at 3$\beta$-position and then the arylsulfonyl group at 23 position to form a compound of formula (XII)

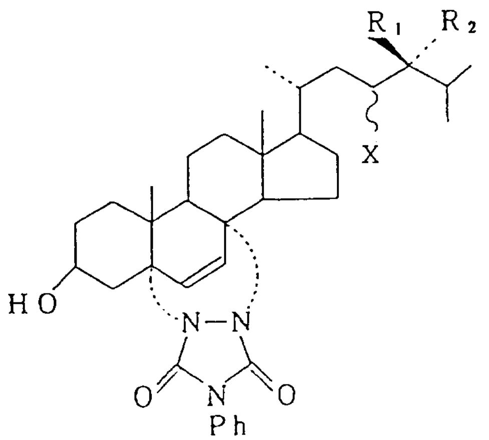

(XII) : $R_1$=Me, $R_2$=H, X=H

;

(c) removing from the resultant compound (XII) the protecting group (PTAD) for 5,7-diene to form a compound of formula (XIV)

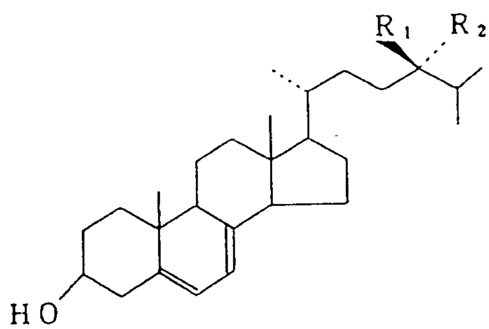

(XIV) : $R_1$=Me, $R_2$=H

;

(d) subjecting the resultant compound (XIV) to irradiation followed by thermal isomerization to form a vitamin $D_7$ compound of formula (XVI)

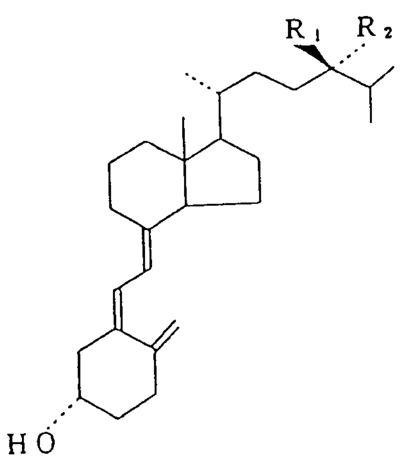

(XVI) : $R_1$=Me, $R_2$=H

;

(e) tosylating the vitamin $D_7$ compound followed by solvolyzing the vitamin $D_7$ tosylate to form a 3,5-cyclovitamin $D_7$ of formula (XVIII)

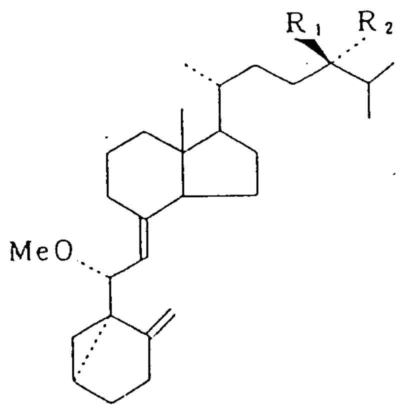

(XVIII) : $R_1$=Me, $R_2$=H

;

(f) oxidizing the 3,5-cyclovitamin $D_7$ to form 1α-hydroxy-3,5-cyclovitamin $D_7$ of formula (XX)

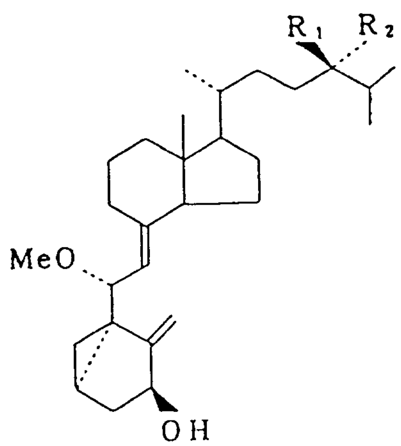

(XX) : $R_1$=Me, $R_2$=H

;

and

(g) sequentially solvolyzing the 1α-hydroxy-3,5-cyclovitamin $D_7$ and subjecting to saponification the 1α-hydroxy vitamin $D_7$ 3β-acetate of formula (XXII) to give 1α-hydroxy vitamin $D_7$.

**8.** A method of preparing a 1α-hydroxy vitamin $D_4$ compound of formula (II)

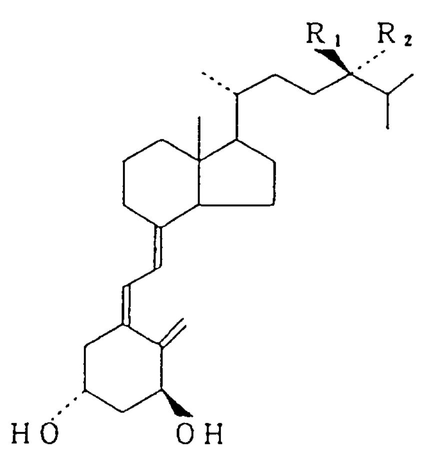

(II) : $R_1$=H, $R_2$=$CH_3$

which comprises:

(a) condensing the 22-halo compound of formula (V)

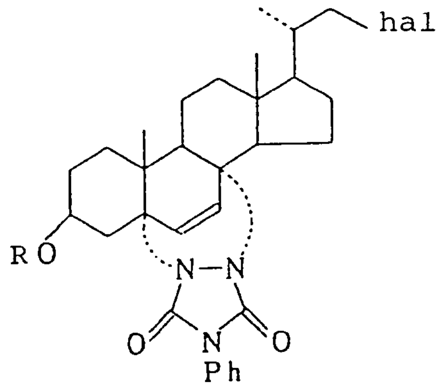

(V)

with a sulfone derivative of formula (VII)

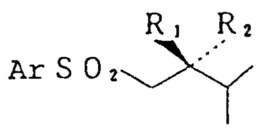

(VII) : $R_1$=H, $R_2$=Me

to form a compound of formula (IX)

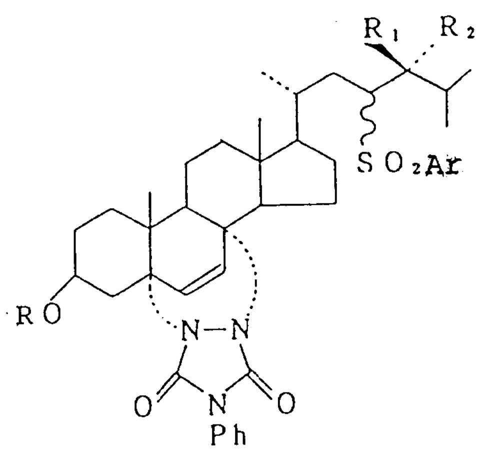

(IX) : $R_1$=H, $R_2$=Me

;

(b) removing from the resultant compound (IX) the protecting group at 3$\beta$-position and then the arylsulfonyl group at 23-position to form a compound of formula (XIII)

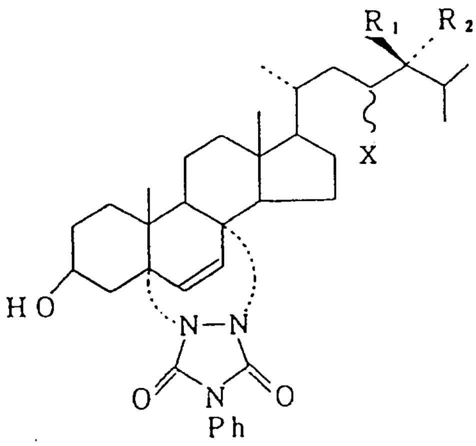

(XIII) : $R_1=H$, $R_2=Me$, X=H

;

(c) removing from the resultant compound (XIII) the protecting group (PTAD) for 5,7-diene to form a compound of formula (XV)

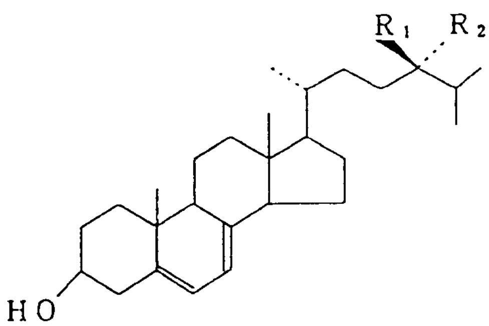

(XV) : $R_1=H$, $R_2=Me$

;

(d) subjecting the resultant compound (XV) to irradiation followed by thermal isomerization to form a vitamin $D_4$ compound of formula (XVII)

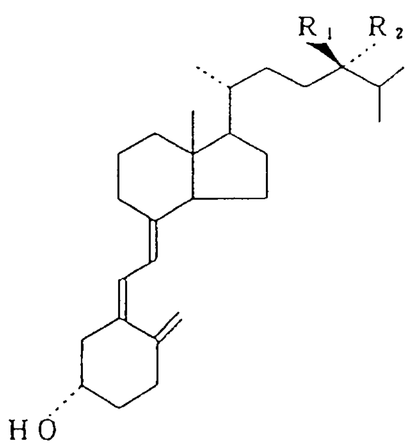

(XVII) : $R_1=H$, $R_2=Me$

;

(e) tosylating the vitamin $D_4$ compound followed by solvolyzing the vitamin $D_4$ tosylate to form a 3,5-cyclovitamin $D_4$ of formula (XIX)

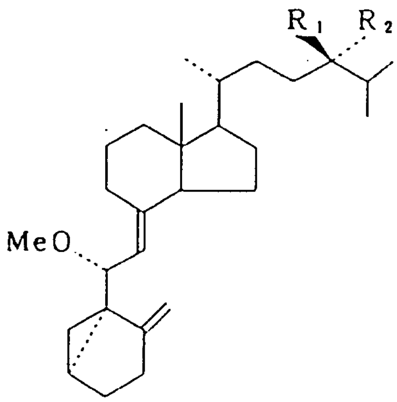

(XIX) : $R_1$=H, $R_2$=Me

;

(f) oxidizing the 3,5-cyclovitamin $D_4$ to form 1α-hydroxy-3,5-cyclovitamin $D_4$ of formula (XXI)

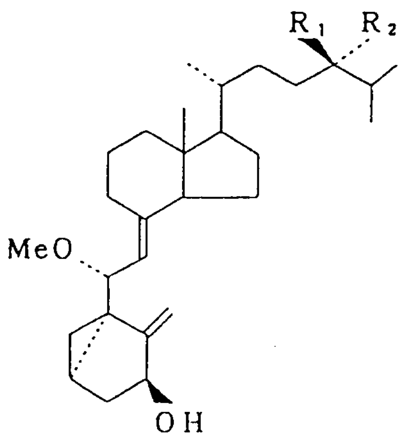

(XXI) : $R_1$=H, $R_2$=Me

;

and
(g) sequentially solvolyzing the 1α-hydroxy-3,5-cyclovitamin $D_4$ and subjecting to saponification the 1α-hydroxy vitamin $D_4$ 3β-acetate of formula (XXIII) to give 1α-hydroxy vitamin $D_4$.

European Patent Office

# EUROPEAN SEARCH REPORT

Application Number

EP 93 10 4592

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | BIOCHIMICA ET BIOPHYSICA ACTA vol. 1091, no. 2, 1991, AMSTERDAM NL pages 188 - 192 F. SATO ET AL 'Biological Activity of 1.alpha.,25-Dihydroxyvitamin D Derivatives - 24-epi-1.alpha,25-Dihydroxyvitamin D2 and 1.alpha.,25-Dihydroxyvitamin D7' * the whole document * --- | 1,4-6 | C07C401/00<br>A61K31/59<br>C07J9/00<br>C07J71/00 |
| A | BULLETIN OF THE CHEMICAL SOCIETY OF JAPAN. vol. 63, no. 8, August 1990, TOKYO JP pages 2233 - 2238 M. TSUJI ET AL 'Synthesis of 22,23-Dihydro-1.alpha.,25-dihydroxyvitamin D2 and Its 24R Epimer, New Vitamin D2 Derivatives' * the whole document * --- | 1,7-8 | |
| A | EP-A-0 390 097 (NISSHIN FLOUR MILLING CO., LTD.) * the whole document * --- | 1 | |
| P,X | WO-A-9 205 130 (LUNAR CORPORATION) * the whole document * ----- | 1,3-6 | TECHNICAL FIELDS SEARCHED (Int. Cl.5 )<br>C07C<br>A61K |

The present search report has been drawn up for all claims

| Place of search | Date of completion of the search | Examiner |
|---|---|---|
| THE HAGUE | 23 JUNE 1993 | WATCHORN P.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)